# EUROPEAN PATENT SPECIFICATION

(11) **EP 3 886 885 B1**
(45) Date of publication and mention of the grant of the patent: **19.04.2023**
(21) Application number: 19828634.6
(22) Date of filing: 12.12.2019
(51) Int. Cl.: A61K 36/898, A61P 1/00

(54) **METHOD FOR PREPARING A COMPOSITION AND USE OF THE COMPOSITION FOR TREATMENT OR PROPHYLAXIS OF DISEASE**
VERFAHREN ZUR HERSTELLUNG EINER ZUSAMMENSETZUNG UND VERWENDUNG DER ZUSAMMENSETZUNG ZUR BEHANDLUNG ODER PROPHYLAXE VON KRANKHEITEN
PROCÉDÉ DE PRÉPARATION D'UNE COMPOSITION ET UTILISATION DE LA COMPOSITION POUR LE TRAITEMENT OU LA PROPHYLAXIE D'UNE MALADIE

(43) Date of publication of application: 06.10.2021
(73) Proprietor: QUANTUM PHARMACEUTICALS SA, 2001 Neuchâtel (CH)
(72) Inventor: DAHLGREN, Atti-La, 2022 Bevaix (CH); DAHLGREN, John, 2024 Marin-Epagnier (CH)
(74) Representative: Fenix Legal KB
(86) International application number: PCT/EP2019/084998
(87) International publication number: WO 2021/115610

(56) References cited:
- WO-A1-2011/093769
- WO-A1-2016/122091
- KR-A- 20090 083 082

## Description

### FIELD OF THE INVENTION

The present invention relates to a composition and the method for preparing the composition of the plant *Cymbidium erythraeum,* suitable to influence gut microbiota composition and enzyme activity and may be used for the treatment or prophylaxis of diseases in humans, in particular for the treatment or prophylaxis of diseases in humans related to an impared or reduced function of gut microbiota and enzyme quinone reductase e.g. due to exposure to endogenous and/or exogenous events including chronological or accelerated aging processes.

### SUMMARY OF THE INVENTION

Disclosed is a method for creating a composition suitable to correct dysbiosis.

A method for preparing a composition suitable for use in the treatment or prophylaxis of disease in humans related to an impaired or reduced function of the gut microbiota and the enzyme quinone reductase obtained from fermented *Cymbidium eyrthraeum* comprising plant material including a plant or plant parts of all wild types, hybrids and cultivars, found in the wild or cultivated in vitro. The method comprises the steps of withering the plant material such as leaves, flowers, stem, seeds, cells, stem cells and/or root including tissues, cells and/or stem cells of the plants or plant parts; macerating mechanically the withered plant material; fermenting the macerating plant material by an enzymatic, bacterial or fungal process; drying and pulverising the fermented plant material; sonicating and macerating the pulverised plant material with solvents; and extracting the macerated plant material.

There is also provided a composition obtained by the claimed method used in the treatment or prophylaxis of disease in humans.

The method for preparing a composition by fermentation of *Cymbidium erythraeum* is removing structural plant components which otherwise hinder the optimal extraction of secondary plant metabolites.

The invention described herein has surprisingly shown to improve the microbial microhabitat and its surroundings in the human gut, providing numerous benefits towards the prevention and treatment of disease and dysbiosis, including boosting the activity of the enzyme quinone oxidase. It does so by providing necessary micro environment in the form of a fermented composition of the plant *Cymbidium erythraeum.*

Additional substances may be added to the composition of the invention, including supernatants of microbial cultures and metabolic factors to aid the development of the beneficial microbiota and materials to aid administration to the target environment.

### BACKGROUND OF THE INVENTION

The ageing process is being recognized by an increasing number of scientists as a disease (1-3). The ageing process has genetic as well as environmental causes. The environmental causes comprise the internal physiological environment of the individual organism and external influences. The internal environment is reflected by the physiological homeostasis, hormonal balance, utilization of nutrients, cellular repair mechanisms and antioxidant status. External environmental ageing factors may be chronic radiation (UV) injury (CRI) from the sunlight, smoking, diet, medication, stress and lifestyle.

Many of these external environmental factors have an accelerating influence on the ageing processes through e.g. formation of free radicals contributing to other age related disturbances like decreased cognitive function (5-9). Accelerated ageing of other organ systems also linked to external influences are gastro-intestinal conditions e.g. Crohn's disease, Ulcerative colitis, Irritable bowel syndrome (IBS) and dysbiosis of the gastrointestinal microbiota, obestiy, metabolic syndrome, liver conditions like NASH ( Non Alcoholic Steatohepatitis), NAFLD (Non Alcoholic Fatty Liver Disease) and Cirrhosis (10-13) which are high risk factors for liver cancer.

Frailty has been defined by Fried et al. as a syndrome of decreased reserve and resistance to stressors, resulting from cumulative declines across multiple physiological systems, causing vulnerability to adverse outcomes. The combine prevalence in a meta-analysis of 2009 showed that 17% of those aged 65 or older in Europe could be considered frail. The frailty phenotype has been used to assess physical frailty by evaluating weight loss, exhaustion, weakness (eg, by handgrip strength), slow walking speed and low physical activity. Several other scores from different scientific and care disciplines exist that include measures of medical, psychological, cognitive, functional and/or social loss. Frailty is linked with an higher risk of health issues with increased risk of disability and decreased quality of life, and the ever larger group of frail elderly is conducive to more direct (for example, medical or physiotherapy consultations, diagnostic procedures, hospitalisations, medication use) and indirect healthcare costs (for example: social / daily support). It is therefore of great importance to find solutions in modifiable factors and preventive strategies.

The gut microbiome, which is the ecosystem of microbes found in the intestine, is a promising target as it is connected to a great number of host functions, is influenced by environmental factors, and disturbances have been reported in the ageing population. Compounds aiming to influence the composition and function of the intestinal microbiome, could improve intestinal health and general well-being of the ageing population.

It is a challenge to try to understand in what way the human intestinal microbiome influence ageing. The microbiome is a very intricate ecology of organisms that changes within the individual over time, which makes age related changes difficult to measure. But there are differences between younger and elderly populations with regard to the microbiome composition. However are these differences just a reflection of a generally ageing physiology and change in diet, or could the microbiome actively drive the ageing process of the host? Model organisms have been used to answer these questions. Changes in microbiota composition precede intestinal and host aging in studies in fruit flies (*Drosophila melanogaster*) and antibiotics increases the lifespan implicating microbes in accelerating ageing. Antibiotics also extend the lifespan of middle-aged killifish but additional transplantation of gut microbes from young killifish extends lifespan further, suggesting a positive effect of microbes associated with young animals. Microbes from old, but not young, mice induce inflammation when added to germ-free mice suggesting that microbes become more harmful to the host with age. These studies implicate broad classes of bacteria, particularly members of the phylum Proteobacteria, as drivers of aging leading to intestinal degradation and inflammation. The nematode *Caenorhabditis elegans* can be linked with single strains of genetically-tractable bacteria, and this simplified system has revealed specific interventions in bacterial metabolism, such as inhibition of bacterial folic acid synthesis, that extend animal lifespan. Transferring this understanding to the human microbiome will need more research but promises to reveal how manipulation of the gut microbiota might be a route to maintain health in old age. Age is used in virtually all studies of cancer epidemiology and is one of the most studied risk factors for cancer. Cancer can be considered an age-related disease because the incidence of most cancers increases with age,rising more rapidly beginning in midlife (14). A key detoxifying enzyme is quinone reductase which is abundant in the liver and the skin. This enzyme exists in two forms NQO1 (EC 1.6.99.2) and NQO2 (EC 1.10.5.1).

The document WO2011/093769A1 discloses an extract of Dionaea muscipula and a composition comprising such an extract for cosmetic treatment of the skin and skin appendages. Such a composition may also contain an extract from Cymbidium erythraeum.

Another document WO2016/122091 A1 relates to a pharmaceutical composition, an external preparation for skin, a cosmetic composition and a health functional food for prevention and treatment of allergic diseases or contact dermatitis, comprising a cymbidium extract as an active ingredient.

Furthermore, the document KR 2009 0083082A relates to a composition for improving skin function, which contains Orchidaceae fermented extract having the effects of inflammation relief or skin barrier improvement.

### Cancer prevention and the role of quinone reductase

Chemoprevention of cancer includes prevention, delay, or reversal of the process of carcinogenesis through ingestion of dietary or pharmaceutical agents (15,16). Recent advances that have defined the cellular and molecular events associated with carcinogenesis, along with a growing body of experimental, epidemiological, and clinical trial data, provide a foundation for relatively new strategies of cancer prevention(1 7,18). One such strategy involves suppression of carcinogen metabolic activation or blocking the formation of ultimate carcinogens (19). In particular, the induction of phase II enzymes can offer protection against toxic and reactive chemical species (20). Many recent studies have shown that elevation of phase II enzymes, such as quinone reductase, correlates with protection against chemical-induced carcinogenesis in animal models, in the stage of promotion as well as initiation (21,22).

### The Gut-Skin axis

Both the skin and the gut are essential organs for the maintenance of our physiological homeostasis as our primary interface with the external environment. They are both highly vascularized and densly innervated with important immune and neuroendocrine functions and linked in role and purpose (23). Research has demonstrated that a close, bidirectional link exists between the skin and the gut, and a number of studies connects gastrointestinal health to skin homeostasis (23,24). Disorders of the gastrointestinal tract are often accompanied by dermatological manifestations and the gut microbiome, seem to play a role in the pathophysiology of many skin disorders (25-27).

In mammals levels of polyamines decrease during the aging process. Reductions in intestinal luminal polyamine levels have been associated with intestinal barrier dysfunction. Probiotics have been shown to increase intestinal lumimal concentrations of polyamines, improve intestinal barrier function, increased mucus secretion and gene expression in older mice resembled those of much younger mice (28).

The visible signs of skin aging and accelerated skin aging is mainly caused by external factors, resulting in a skin that appears older compared to individuals of the same chronological age who are not exposed in the same degree to the causative factors. The Chronic Radiation Injury (CRI) by cumulative UV exposure leads to the formation of micro-scars in the dermal matrix by the activation of reactive oxygen species (ROS) which effectively can be neutralized by the Phase II detoxifying enzymes quinone oxidase present in the skin.

There is a need for a solution to the urgent problem of addressing the multifaceted consequences of aging linked to the gut microbiota through a new approach using a novel composition.

### REFERENCES

1. Bulterijs S, Hull RS, Björk VCE, and Roy A. It is time to classify biological aging as a disease Front Genet. 2015; 6: 205. doi: 10.3389/fgene.2015.00205
2. Mutaz Musa, Aging, Just Another Disease, The Scientist, November 2016
3. Timothy V. Gladyshev and Vadim N. Gladyshev. A Disease or not a Disease? Aging as a Pathology, Trends Mol Med. 2016 Dec; 22(12): 995-996.
4. Commenges, D., Scotet, V., Renaud, S., Jacqmin-Gadda, H., Barberger-Gateau, P., & Dartigues, J.-F. (2000). Intake of flavonoids and risk of dementia. European Journal of Epidemiology, 16, 357-363.
5. Dai, Q., Borenstein, A.R., Wu, Y., Jackson, J.C., & Larson, E.B. (2006). Fruit and vegetable juices and Alzheimer's disease: The Kame Project. American Journal of Medicine, 119, 751-759.
6. Engelhart, M.J., Geerlings, M.I., Ruitenber, A., van Swieten, J.C., Hofman, A., Witteman, J.C.M., et al. (2002). Dietary intake of antioxidants and risk of Alzheimer disease. Journal of the American Medical Association, 287, 3223- 3229.
7. Morris, M.C., Evans, D.A., Bienias, J.L., Tangney, C.C., Bennett, D.A., Aggarwal, N., et al. (2002). Dietary intake of antioxidant nutrients and the risk of incident Alzheimer disease in a biracial community study. Journal of the American Medical Association, 287, 3230-3237.
8. Solfrizzi, V., Panza, F., & Capurso, A. (2003). The role of diet in cognitive decline.Journal of Neural Transmission, 110, 95-110.
9. Molodecky NA, Kaplan GG, Environmental risk factors for Inflammatory Bowel Disease, gastroenterol hepatol (N Y). 2010; 6(5): 339-346.
10. Marynowski M, Likonska A, Zatorski H and Fichna J. Role of environmental pollution in irritable bowel syndrome. World J gastroenterol. 2015; 21 (40): 11371-11378.
11. Frasca D, Blomberg B and Paganelli R. Ageing, Obesity and Inflammatory Age-related Diseases. Front.lmmunol. 2017, 8:1745
12. Saltzman E T, Palacios T, Thomsen M and Vietta L. Intestinal Microbiome Shifts, dysbiosis, Inflammation and Non-alcoholic Fatty Liver Disease. Front Microbiol 2018; 9:61
13. White M, Holman D M, Boehm J E et al. Age and Cancer Risk. Am J Prev Med. 2014 Mar; 46(3 0 1): S7-15. doi: 10.1016/j.amepre.2013.10.029
14. Kohar I, Baca M, Suarna C, Stocker R, Southwell-Keely PT (Aug 1995). "Is alpha-tocopherol a reservoir for alpha-tocopheryl hydroquinone?". Free Radical Biology & Medicine. 19 (2): 197-207. doi:10.1016/0891-5849(95)00010-U
15. Sporn MB, Newton DL. Fed Proc. 1979;38:2528-2534.
16. Hong WK, Sporn MB. Science. 1997;278:1073-1077.
17. KelloffGJ, Sigman CC, Greenwald P. Eur J Cancer. 1999;35:1755-1762.
18. Kensler TW. Environ Health Perspect. 1997;105(Suppl 4):965-970.
19. Wattenberg LW. Cancer Res. 1985;45:1-8.
20. BegleiterA, Leith MK, Curphey TJ, Doherty GP. Oncol Res. 1997;9:371-382.
21. Song LL, Kosmeder JW, II, Lee SK, Gerhäuser C, Lantvit D, Moon RC, Moriarty RM, Pezzuto JM. Cancer Res. 1999;59:578-585.
22. Boone CW, Steele VE, Kelloff GJ. Mutat Res. 1992;267:251-255.
23. O'Neill, C. A., Monteleone, G., McLaughlin, J. T., and Paus, R. (2016). The gut-skin axis in health and disease: a paradigm with therapeutic implications. Bioessays 38, 1167-1176. doi: 10.1002/bies.201600008
24. Levkovich, T., Poutahidis, T., Smillie, C., Varian, B. J., Ibrahim, Y. M., Lakritz, J. R., et al. (2013). Probiotic bacteria induce a 'glow of health'. PLoS One 8:e53867. doi: 10.1371/journal.pone.0053867
25. Shah, K. R., Boland, C. R., Patel, M., Thrash, B., and Menter, A. (2013). Cutaneous manifestations of gastrointestinal disease: part I. JAAD 68, 189.e1-189.e21. doi: 10.1016/j.jaad.2012.10.037
26. Thrash, B., Patel, M., Shah, K. R., Boland, C. R., and Menter, A. (2013). Cutaneous manifestations of gastrointestinal disease: part II. JAAD 68, 211.e1-211.e33. doi: 10.1016/j.jaad.2012.10.036
27. Gloster, H. M., Gebauer, L. E., and Mistur, R. L. (2016). "Cutaneous manifestations of gastrointestinal disease," in Absolute Dermatology Review, eds H. M. Gloster, L. E. Gebauer, and R. L. Mistur (Cham: springer), doi: 10.1007/978-3-319-03218-4_48
28. Matsumoto M, Kurihara S, Kibe R et al. Longevity in mice is promoted by probiotic induced suppression of colonic senescence dependent on Up-regulation of gut bacterial polyamine production. 2011, PLOS on,e vol 6, (8), 1-9.

### DETAILED DESCRIPTION

### First aspect

A method to prepare a composition from *Cymbidium eyrthraeum* including all wild types, hybrids and cultivars, found in the wild or in cultivation around the world for example: The Kingdom of Bhutan, The United States of America, India, The Peoples Republic of China, South East Asia, tropical or subtropical regions of the world, indoor or outdoor cultivation in all countries and territories of the world, characterized in that plants or plant parts are processed, and said composition is suitable for use in the treatment or prophylaxis of disease in humans related to an impaired or reduced function of the gut microbiota and the enzyme quinone reductase.

The methods to prepare the composition according to the invention are described in the following examples:

### Example 1.

According to one embodiment of the invention where the composition is obtained from fermenting *Cymbidium erythraeum,* by a process comprising the following non-limiting procedures, using the whole plant, or any part thereof e.g. the leaves, flowers, buds, stem, seeds, cells, and or root of the plant including tissues, cells and/or stem cells obtained from the plant or cultivated in vitro:
1. The plant material is obtained from a growing plant or cultured cells. The plant material are cut in 0.1-1 cm pieces.
2. Withering.

The plant parts are left for a period of time to wilt to allow for a gradual onset of enzymatic oxidation. The process also removes excess water from the plant material and is important in promoting the breakdown of proteins. The humidity should be less than 70% most preferably less than 30% and the temperature should not exceed 45 ° C. This process takes from 0.1 h - 12 hours, most preferred 2 hours.

### 3. Mechanical maceration of the plant material.

The mechanical maceration is conducted using appropriate machinery. The plant parts are disrupted mechanically in the tumbler which allows co-mingling of oxidative enzymes. This process take from 0.1h - 12 h, most preferred 1 h. 4. Fermentation (e.g. enzymatic, bacterial and or fungal). The fermentation of *Cymbidium erythraeum* removes structural plant components which otherwise hinder the optimal extraction of secondary plant metabolites.

The fermentation step is a solid or liquid fermentation, preferably solid fermentation. The solid fermentation is performed with one or more of the following microorganisms: fungi, bacteria, yeast, archea, algae, the most preferred organisms are bacteria.

The bacteria used in the fermentation step are preferably one or more of the following: genus Pedicoccus, genus Lactococcus, genus Lactobacillus (e.g. *L.plantarum, L. brevis, L. casei, L. rhamnosus, L. curvatus, L. gasseri, L. pentosus*, *L. acidophilus*)*,* Bacillus pumilus, Bacillus subtilis, Trichoderma reesei, *E faecium, P.acidilactici and P. pentosaceus.*

The microorganisms used for the fermentation step are in a 1:1 - 1:1000 ratio, most preferable a 1:50 ratio microbe to Cymbidium plant material.

The fermentation time takes 0.1h - 168 h, most preferred time 12-24 h.

### 5. Drying

Using a low temperature vacuum dryer. The temperature should not exceed 35°C.

### 6. Pulverisation

Using a cryogenic liquid nitrogen grinder. 90% of the powder should pass through a 212 µm sieve.

### 7. Sonicated with solvents

The plant material obtained after fermentation, drying and pulverization is mixed with a solvent where the non-limiting examples of solvents used are: water, super-critical CO₂ (Carbon dioxide), alcohols, ethanol, methanol, dichlor methane (DCM), acetyl acetate, acetone, vegetable oils etc. Most preferred solvent is 95% ethanol and sonicated for 1 min - 1440 min, most preferably for 30-45 minutes.

### 8. Maceration with solvents

The plant material after the sonication step is macerated in 95% ethanol for 1h-168h, most preferably for 72 h at room temperature.

Agitation is done every 12 h for 15 minutes.

### 9. Extraction

The extraction step uses a suitable solvent selected from the group of solvents consisting of: acetone, carbon dioxide, isopropanol or t-butanol and esters of acetic acid, preferably ethyl acetate, alcohols, ethanol, isopropyl alcohol, acetyl acetate, acetone, vegetable oil, etc)

According to one embodiment of the invention the extraction procedure would be:
(a) placing *Cymbidium erythraeum* material in a suitable solvent, preferably 95% ethanol in water to achieve extraction of the plant material;
(b) separating the liquid and solid contents;
(c) recovering a first fraction from the liquid contents by filtration of the solvent present in the liquid contents;
(d) placing the solid contents in an organic solvent selected from the group of solvents consisting of: acetone, carbon dioxide, isopropanol or t-butanol and esters of acetic acid, preferably ethyl acetate to achieve extraction of the remaining fraction from the plant material;
(e) separating the liquid and solid contents;
(f) recovering a second fraction by filtration of the solvent from the liquid contents under reduced pressure;
(g) recovering the solid contents,
   and
h) lyophilize the extract.

The amount of free phenolic and conjugated phenolic compounds is estimated by the Folin-Ciocalteau method and should be between 1 µg/g - 100000 µg/g dry weight of plant material (DW), most preferably between 100 µg/g -5000 µg/g DW The amount of proteins should be between 1 µg/g - 1000 µg/g, most preferably between 25 µg/g - 200 µg/g

### Example 2

After harvest of flowers and flower buds and cutting the plant material in small 0.5-1 cm pieces or by grinding the fresh plant material, fermentation takes place in a solid state with *Bacillus pumilus* in a ratio 1:60 parts bacteria and plant material repectively.

The powdered fermented flowers and flower buds ( 500 grams) were steeped in 50L of Ethanol for 24 h at room temperature. The mixture was sonicated for the first 30 minutes after which maceration took place and aggitaion every 3 hours. The extract was filtered and concentrated under vacuum at 40°C using a rotary evaporator. The dried crude ethanol extract was fractioned and dissolved in methanol and mixed with 40 Liter of 4 M NaOH by constantly stirring at 60°C for 4 h. The hydrolyzed solution was centrifuged for 10 min at 5000 rpm and the supernatant was filtered. The resulting solution was acidified to pH 1.5 with 6 M HCL. Subsequently it was centrifuged for 10 min at 5000 rpm and filtered afterwards. The filtrate was extracted with 40 L of ethyl acetate. The extract was evaporated to dryness by a rotary evaporator at 30° C.

The amount of free phenolic and conjugated phenolic compounds were estimated by the Folin-Ciocalteau method and found to be 1400 µg/g DW.

### Example 3

After harvest of flowers and flower buds and cutting the plant material in small 0.5-1 cm pieces or by grinding the fresh plant material, fermentation takes place in a solid state with *Bacillus pumilus and Lactococcus lactis (50*/*50)* in a ratio 1:50 parts bacteria and plant material repectively.

The powdered fermented flowers and flower buds (500 grams) were steeped in 50L of protein extracto 24 h at room temperature. The mixture was sonicated for the first 30 minutes after which maceration took place and aggitaion every 3 hours. The extract was filtered and concentrated under vacuum at 10°C using a rotary evaporator. Subsequently it was centrifuged for 20 min at 12000 rpm and filtered afterwards. The extract was evaporated to dryness by a rotary evaporator at 30° C.

The amount of protein was found to be 75 µg/g DW.

### Second aspect

A composition obtained by a method of the first aspect.

A second aspect of the invention relates to a composition obtained by a method of the first aspect comprising a fermented *Cymbidium eyrthraeum* for treatment or prophylaxis of diseases in humans, in particular for the treatment or prophylaxis of diseases related to the gut-skin axis, gut microbiota dysbiosis, and impaired or reduced function of the enzyme quinone reductase NQO1 (EC 1.6.99.2) and NQO2 (EC 1.10.5.1), e.g. due to exposure to endogenous and/or exogenous events including chronological or accelerated aging processes.

Dosage for the treatment or profylaxis with the invention, 0.01 mg to 10000 mg per day to a human, preferably 1- 100 mg of 100% product per day is recommended depending on the disorder and the severity of the disorder.

The composition according to the second aspect may be administered to a human in any suitable galenic form such as the following non-limiting examples:, drinks, syrups, capsules, tablets, enteric coated tablets, enteric coated capsules, suppositories, enemas .

A further embodiment of the invention may combine the composition according to the second aspect with any other substances and components which facilitates the administration or supports the effect of the invention.

The invention may contain other bioactive compounds such as: bioactive carbohydrates, lipids, amino acids, proteins, peptides, oligo-elements, enzymes, plant tissue factors, enzyme inhibitors, flavonoids, thylacoids, polyphenols, and xanthophylls.

Further non-limiting examples of active ingredients and plant extracts that may be added to the composition are:
Ascorbic acid, astaxanthin, haematococcus algae, probiotic substances, prebiotic substances, postbiotic substances, Omega-3 fatty acids, fishoil, plankton oil,
Spirulina platensis extract, phycocyanin, Fucoxanthin, , coenzyme Q10, gluathion, amino acids e.g. cystein, vitamins, tocopherol, lutein, lycopene, nicotine amide, polyphenols.

### Third Aspect

The use of the first aspect or a composition according to the second aspect for treatment and prophylaxis of diseases in humans.

The obtained composition has surprisingly shown to improve the microbial microhabitat and its surroundings in the human gut, providing numerous benefits towards the prevention and treatment of diseases and dysbiosis.

According to one embodiment, the present invention is suitable to influence gut microbiota composition and enzyme activity and may be used for the treatment or prophylaxis of diseases in humans, in particular for the treatment or prophylaxis of diseases related to the gut-skin axis, gut microbiota dysbiosis, and impaired or reduced function of the enzyme quinone reductase NQO1 (EC 1.6.99.2) and NQO2 (EC 1.10.5.1) , e.g. due to exposure to endogenous and/or exogenous events including chronological or accelerated aging processes.

According to another embodiment, the present invention has been shown to influence the composition and function of the intestinal microbiome, towards a less dysbiotic and more young gut microbiota composition.

Examples of gastro-intestinal conditions linked to gut microbiota dysbiosis or aged microbiota are e.g. Crohn's disease, Ulcerative colitis, Irritable bowel syndrome (IBS) and dysbiosis of the gastrointestinal microbiota, obestiy, metabolic syndrome, liver conditions like NASH ( Non Alcoholic Steatohepatitis), NAFLD ( Non Alcoholic Fatty Liver Disease) and Cirrhosis which are high risk factors for liver cancer.

### Example 4

An 86 year old Caucasian lady living on a western diet, was complaining about constipation. Upon gut microbiota analysis is was show she had an increased proportion of the phylum Proteobacter and decrease in the phylum Firmicutes. She also had low proportion of Bifidobacteria a typical finding in elderly individuals. She was given 2 hard gelatin capsules per day containing 200 mg of the composition from example 1. After 2 weeks the proportion of Bifidobacteria had significantly increased and the Proteobacter phylum and Firmucutes to resemble that of younger individuals. The constipation problem was resolved, and her general health improved.

According to another embodiment, the obtained composition has surprisingly shown to improve the impaired or reduced function of quinone reductase NQO1 (EC 1.6.99.2) and NQO2 (EC 1.10.5.1), a key detoxifying Phase II enzyme which is abundant in the liver and the skin. Chemoprevention of cancer includes prevention, delay, or reversal of the process of carcinogenesis through ingestion of dietary or pharmaceutical agents. In particular, the induction of phase II enzymes can offer protection against toxic and reactive chemical species. Many recent studies have shown that elevation of Phase II enzymes, such as quinone reductase , correlates with protection against chemical-induced carcinogenesis in animal models, in the stage of promotion as well as initiation.

### Example 5.

Use of the invention to boost the activity of the enzyme quinone reductase. Quinone reductase is a phase II enzyme (detoxification enzyme) present in large quantities in the liver and the skin. This enzyme can detoxify a wide range of quinones produced by the cellular respiration.

At a concentration of 20µg/ mL in an *in vitro* trial, the composition of the invention doubled the activity of the enzyme quinone reductase.

### Example 6.

Use of the invention in hepatocarcinoma cell line *in vitro.*

Hepatocellular carcinoma (HCC) is the most common type of primary liver cancer in adults, and is the most common cause of death in people with cirrhosis. The study was performed *in vitro* with the liver cancer cell line (Hepa1c1c7-ATCC). The results revealed that an extract according to the invention displayed anticancer activity at 20µg/mL where only 30% of the tumor cells survived.

### Fourth Aspect

A fourth aspect of the invention relates to the first aspect or a composition of the second and third aspect and/or herein described embodiments thereof, for use in the treatment or prophylaxis of a condition related to chronological, premature and/or accelerated aging.

The obtained composition has surprisingly shown to improve skin conditions due to an improvement of the gut-skin axis, by restoring the eubiosis of the gut microbiota and boost the activity of the enzyme quinone reductase.

The extract and the composition according to the invention may be used against accelerated or chronological aging changes especially aging changes of the skin such as wrinkles, loss of firmness and elasticit, increased sebum production, and increased melanin pigmentation.

### Fifth Aspect

The use of the first aspect or a composition according to the second aspect as oral cosmetic (nutricosmetic) for improving the appearance of the skin in humans.

A fifth aspect of the invention relates to the first aspect of the invention or a composition of the second aspect and/or herein described embodiments thereof, for use in the oral cosmetic treatment (nutricosmetic) to improve the visual aspect of human skin, e.g. for treatment of skin changes due to accelerated or chronological aging e.g. wrinkles, loss of firmness and elasticity, and increased pigmentation especially melanin pigmentation and age spots.

### Examples:

The following examples are describing suitable possible relevant embodiments of the present invention.

### Example 7. Hard gelatine capsule (Bovine, Fish or Vegetarian, Kosher/Hallal certified).

A hard gelatine capsule size 2, containing:

| | |
|---|---|
| Dry and powdered Cymbiol^{®} extract | 250 mg |

### Example 8. Hard gelatine capsule (Bovine, Fish or Vegetarian, Kosher/Hallal certified).

A hard gelatine capsule size 0, containing:

| | |
|---|---|
| Dry and powdered Cymbidium erythraeum extract | 250 mg |
| Dry and powdered DionAct^{®} extract | 250 mg |
| Zeatin | 1 mg |

### Example 9. Soft gelatine capsule

A soft gelatine capsule (Bovine or Fish Hallal/Kosher certified gelatine) size Oval 10, is filled according to industry standards with the following composition:

| | |
|---|---|
| Lipid fraction (min 50% in Omega 3 (EPA and DHA)) | 300 mg |
| Marine protein complex | 220 mg |
| Vitamin E | 16 mg |
| Lutein | 15 mg |
| Lycopene | 2.5 mg |
| Astaxanthin | 4 mg |
| Fucoxanthin | 1 mg |
| Cymbidium erythraeum extract | 20 mg |
| Grape (*Vitis vinifera*) stem cell extract | 20 mg |
| Soy lecithin | 30 mg |

### Example 10. Hard gelatine capsule (Bovine, Fish or Vegetarian, Kosher/Hallal certified).

A hard gelatine capsule size 1, with the following composition:

| | |
|---|---|
| Cymbidium erythraeum extract | 10 mg |
| DionAct^{®} extract | 10 mg |
| Phycocyanin | 5 mg |
| Vitamin C | 50 mg |
| Marin Collagen powder | 100 mg |
| Exipients | q.s. |

### Example 11. Tablet 500 mg

A tablet manufactured according to the industry standards with the following composition:

| | |
|---|---|
| Cymbidium erythraeum extract | 25 mg |
| Probiotic bacteria | 200 mg |
| Cystein | 250 mg |
| Vitamin B6 | 1 mg |
| Magnesium stearate | q.s. |

### Example 12. Medicinal diet drink 15 ml vial

A medicinal diet drink with the following composition:

| | |
|---|---|
| Cymbiol^{®} extract | 10 mg |
| *Moringa oleifera* extract | 300 mg |
| Marin Collagen | 400 mg |
| Hyaluronic acid | 50 mg |
| Xylitol (sweetener), natural aroma | q.s. |
| Ad Water (aqua) to | 15 ml |

### Example 13. Suppository 250 mg

A suppository, made according to industry standards (Eur. Ph) with the following composition:

| | |
|---|---|
| Cymbiol^{®} extract | 50 mg |
| Probiotic bacteria | 100 mg |
| L-Cystein | 25 mg |
| *Coffea arabica* extract | 25 mg |
| Phycocyanin | 10 mg |
| Excipients | q.s. |

### Example 14. Enteric coated tablet 500 mg,

An entero-coated tablet with the following composition:

| | |
|---|---|
| Cymbiol^{®} extract | 50 mg |
| DionAct^{®} extract | 50 mg |
| Carnitine | 100 mg |
| 1-4,1-6 beta glucan from *Saccharomyces cerevisiae* | 25 mg |
| Probiotic bacteria | 100 mg |
| Vitamin B2 | 2 mg |
| Exipients | q.s. |

### Examples of use of the invention in Human subjects

### Example 15.

Target patients are ≥18 years, and a confirmed body age, and/or face age, and /or brain function age exceeding the accepted benchmark values established by objective testing compared to the patient's chronological age and degree of frailty. These benchmark values can be established by different methods and tests e.g. assessing the patient's brain age by calculating reaction and memory testing, their body age by assessing their cardiovascular fitness and muscle strength, gut microbiota composition, skin microbiota composition, poly amine contents in feces and blood is determined, systemic inflammatory markers, liver function tests and their faces measured by a devise which analyses the appearance of skin, pores, wrinkles and sun damage.

Patients receive one capsule per day of the invention according to Example 10 for a period of 3 months or longer.

### Example 16.

A 3 months placebo controlled randomized study including 20 subjects given either a topical cream containing 1% or 10% cymbidium extract or an Oral capsule according to Example 9 or a placebo capsule without any active compound. Five subjects in each group.

The skin quality was assessed by an expert panel before and after the treatment and an ultrasound measurement of skin epidermis+dermis thickness in mm (DermaScan^{®}C device Cortex Technology, Denmark) was determined.

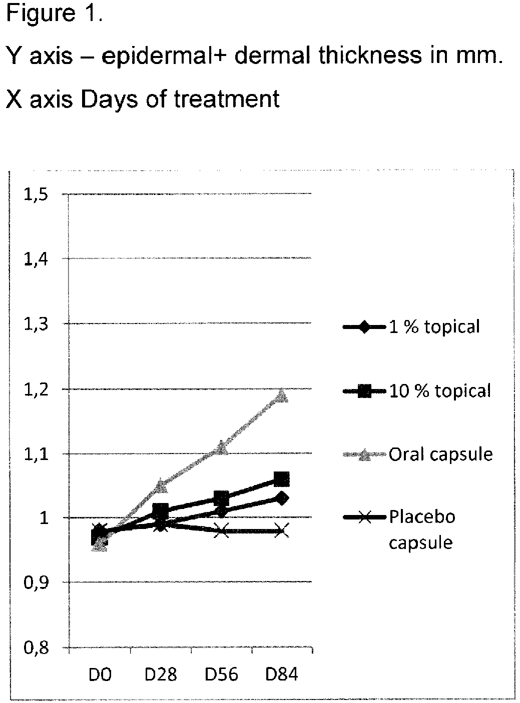

### Oral

| | 1 % topical | 10 % topical | capsule | Placebo capsule |
|---|---|---|---|---|
| D0 | 0.98 | 0.97 | 0.96 | 0.98 |
| D28 | 0.99 | 1.01 | 1.05 | 0.99 |
| D56 | 1.01 | 1.03 | 1.11 | 0.98 |
| D84 | 1.03 | 1.06 | 1.19 | 0.98 |

The results showed unexpectedly a significantly better improvement in this parameter using the fermented Cymbidium extract according to the invention orally as compared with the topical treatments.

### Example 17.

A four months randomized double-blind controlled study including 40 subjects where the supplement according to example 8 was tested against placebo for 3 months followed by a one- month supplement free period for both groups to assess lasting effects. Efficacy measurements included skin surface evaluation, ultrasound measurement of the sun exposed and protected are of the skin and photographic assessment. All investigated parameters showed a continuous and significant improvement in the active group during the 3 months of supplementation as compared to placebo. Ultrasound measurements showed an increase in dermis density of up to 78% in the active group.

### Example 18.

A 44 year old woman of Asian origin noticed a gradual overall loss of skin elasticity and firmness of her skin, in particular in the face and bust. Around the eyes fine lines 'crow's feet' were visible. She started to use the invention (Example 2) orally once per day. Already after a few weeks she noticed a strong lifting effect. During the cause of one month she noticed a gradual improvement of the skin quality, the skin looked more youthful and radiant. And after 3 months her skin felt more firm and she noticed that the fine lines around her eyes were markedly diminished.

### Example 19.

A 24 year old Asian woman was suffering since her mid-teens from oily and acne prone skin on her face and shoulders. Her skin pores were enlarged and often blocked by comedones (so called black heads and white heads). She started to use the invention according to example 6. After two weeks of use the acne started to clear up, the skin was markedly less oily, and the comedones started to diminish. After 4 weeks the skin had cleared up, pores were smaller and previous acne scars were less prominent.

### Example 20.

An Asian woman (37 years) spending a lot of time outdoors in south-east Asia, were suffering from irregular melanin skin coloration caused by repeated strong exposure to sun radiation. She consulted a skin specialist and started to use the invention (Example 9). Already after four weeks usage the skin tone become lighter measured by an objective skin tone scale and the overall skin tone become more even.

### Example 21.

A 37 year old Caucasian woman mother of two children, suffered from so called orange peel skin or cellulites (gynecoid lipodystrophy) over both buttocks and hips. Furthermore she suffered from stretch marks over her stomach after two pregnancies. She started using capsules according to the invention (Example 10) for 8 weeks. After this period a remarkable improvement of the cellulites and the stretch marks could be observed. The skin was firmer, smoother, more elastic and had a more youthful appearance.

### Example 22.

5 patients presenting with Irritable bowel syndrome (Rome IV criteria) was given a suppository according to the invention (Example 7) and 5 was given a placebo, for 4 weeks inserted rectally. The IBS-SSS decreased significantly from over 300 to less than 100 in the group using the invention , no significant change could be observed in the placebo group. The biodiversity in the group using the invention was significantly increased.

### Example 23.

A double blind study with a supplement prepared according to example 7, was given 2 times daily to the active group (5 persons) and the placebo received a similar looking supplement only containing an inactive substance. The study was preceded by randomization and the controls were matching in BMI,age, weight, and lifestyle habits (diet and exercise). The study was conducted over a period of 3 months. Body compositions were measured at the beginning of the trial Day 0, Day 28, Day 56 and Day 84. There was a significant ( p<0.05) decrease in visceral fat in the active group as compared to placebo after 3 months of supplementation in the active group as compared to placebo.

## Claims

1. A method for preparing a composition suitable for use in the treatment or prophylaxis of disease in humans related to an impaired or reduced function of the gut microbiota and the enzyme quinone reductase obtained from fermented *Cymbidium eyrthraeum* comprising plant material including a plant or plant parts of all wild types, hybrids and cultivars, found in the wild or cultivated in vitro **characterized in that** the method comprises the steps of:
- withering the plant material such as leaves, flowers, stem, seeds, cells, stem cells and/or root including tissues, cells and/or stem cells of the plants or plant parts,
- macerating mechanically the withered plant material,
- fermenting the macerating plant material by an enzymatic, bacterial or fungal process,
- drying and pulverising the fermented plant material,
- sonicating and macerating the pulverised plant material with solvents, and
- extracting the macerated plant material.

2. The method according to claim 1, wherein the fermentation step is a solid or liquid fermentation, preferably solid fermentation.

3. The method according to any of claims 1 and 2, wherein the solid fermentation is performed with one or more of the following microorganisms: fungi, bacteria, yeast, archea, algae.

4. The method according to any of claims 1-3 where the bacteria used in the fermentation step are preferably one or more of the following: genus Pedicoccus, genus Lactococcus, genus Lactobacillus (e.g. *L.plantarum, L. brevis, L. casei, L. rhamnosus, L. curvatus, L. gasseri, L. pentoses*, L. acidophilus), Bacillus pumilus, Bacillus subtilis, Trichoderma reesei, E faecium, P.acidilactici and P. pentosaceus.

5. The method according to any of claims 1-4 where the microorganisms used for the fermentation are in a 1:1 - 1 :1000 ratio, most preferable a 1:50 ratio microbe to Cymbidium plant material.

6. The method according to any of claims 1-5, wherein the fermentation time takes between 0.1 hour and 168 hours, preferably 12-24 hours.

7. The method according to any of claims 1-6 where the solvents used are one or more of any of water, super-critical CO₂ , alcohols, ethanol, methanol, dichlor methane (DCM), acetyl acetate, acetone and vegetable oils.

8. The method according to any of claims 1-7 where in the step of sonication, an ultrasound device is used for 1 min - 1440 min, preferably for 30-45 minutes.

9. The method according to any of claims 1-8 where the plant material after the sonication step is macerated in 95% ethanol for 1h- 168h, preferably for 72 h at room temperature.

10. The method according to any of claims 1-9 whereby the extraction step uses a suitable solvent selected from the group of solvents comprising acetone, carbon dioxide, isopropanol or t-butanol and esters of acetic acid, preferably ethyl acetate, alcohols, ethanol, isopropyl alcohol, acetyl acetate, acetone, vegetable oil, and further comprises the steps of:
(a) placing *Cymbidium erythraeum* material in a suitable solvent, preferably 95% ethanol in water to achieve extraction of the plant material;
(b) separating the liquid and solid contents;
(c) recovering a first fraction from the liquid contents by filtration of the solvent present in the liquid contents;
(d) placing the solid contents in an organic solvent selected from the group of solvents comprising acetone, carbon dioxide, isopropanol or t-butanol and esters of acetic acid, preferably ethyl acetate to achieve extraction of the remaining fraction from the plant material;
(e) separating the liquid and solid contents;
(f) recovering a second fraction by filtration of the solvent from the liquid contents under reduced pressure;
(g) recovering the solid contents, and
h) lyophilized.

11. A composition obtained by the method according to any of claims 1-10, **characterized in that** it is used in the treatment or prophylaxis of disease in humans.

12. The composition according to claim 11 for use in the treatment or prophylaxis of gastro intestinal disorder, cancer or skin disorders in humans related to an impaired or reduced function of the gut microbiota and the enzyme quinone reductase NOO1 and NQO2.

13. The composition according to any of claims 11-12 for use in the treatment or prophylaxis of gut microbiota dysbiosis.

14. The composition according to any of claims 11-13 for use in the treatment or prophylaxis of gastro intestinal disorders related to gut microbiota dysbiosis such as Crohn's disease, Ulcerative colitis, Irritable bowel syndrome, dysbiosis of the gastrointestinal microbiota, obestiy, metabolic syndrome, liver conditions like Non Alcoholic Steatohepatitis, Non Alcoholic Fatty Liver Disease and Cirrhosis.

15. The composition according to any of claims 11-13 for use in the treatment or prophylaxis of cancer particularly hepatocellular carcinoma to boost the activity of quinine reductase NOO1 and NQO2.

16. The composition according to any of claims 11-13 for use in the treatment or prophylaxis of skin disorders related to gut microbiota dysbiosis and the gut-skin axis, such as Acne vulgaris, Rosacea.

17. The composition according to any of claims 11-12 for use in the treatment or prophylaxis of skin disorders related to decreased or impaired function of the enzyme quinine reductase NOO1 and NQO2, specifically for accelerated or chronological aging changes of the skin such as wrinkles, loss of firmness and elasticity, increased sebum production, and increased melanin pigmentation.

18. The composition for use according to any of claims 11-17, wherein the range of the amount distributed for daily dose of the treatment or profylaxis is between 0.01 mg to 10000 mg, preferably between 1 mg -100 mg of 100% product to a human, depending on the disorder and its severity.

19. The composition for use according to any of claims 11-17, wherein the composition is administered to a human in any suitable galenic form such as drinks, syrups, capsules, tablets, enteric coated tablets, enteric coated capsules, suppositories, enemas.

20. The composition for use according to any of claims 11-19, wherein the composition further comprises other bioactive compounds such as: bioactive carbohydrates, lipids, amino acids, proteins, peptides, oligo-elements, enzymes, plant tissue factors, enzyme inhibitors, flavonoids, thylacoids, polyphenols, and xanthophylls.

21. The composition for use to any of claims 11-20 wherein the composition further comprises active ingredients and plant extract such as ascorbic acid, astaxanthin, haematococcus algae, probiotic substances, prebiotic substances, postbiotic substances, Omega-3 fatty acids, fishoil, plankton oil, spirulina platensis extract, phycocyanin, Fucoxanthin, coenzyme Q10, gluathion, amino acids e.g. cystein, vitamins, tocopherol, lutein, lycopene, nicotine amide, polyphenols.

## Patentansprüche

1. Verfahren zur Herstellung einer Zusammensetzung, die zur Verwendung bei der Behandlung oder Prophylaxe einer Erkrankung bei Menschen im Zusammenhang mit einer beeinträchtigten oder reduzierten Funktion der Darmmikrobiota und des Enzyms Chinonreduktase geeignet ist, die aus fermentiertem *Cymbidium eyrthraeum* erhalten wird, umfassend Pflanzenmaterial, einschließlich einer Pflanze oder Pflanzenteilen aller Wildtypen, Hybriden und Sorten, die in freier Wildbahn gefunden oder in vitro kultiviert werden, **dadurch gekennzeichnet, dass** das Verfahren die Schritte umfasst:
- Verwelken des Pflanzenmaterials wie Blätter, Blüten, Stängel, Samen, Zellen, Stammzellen und/oder Wurzel einschließlich Gewebe, Zellen und/oder Stammzellen der Pflanzen oder Pflanzenteile,
- mechanisches Aufweichen des verwelkten Pflanzenmaterials,
- Fermentieren des mazerierenden Pflanzenmaterials durch einen enzymatischen, bakteriellen oder pilzlichen Prozess,
- Trocknen und Pulverisieren des fermentierten Pflanzenmaterials,
- Beschallen und Mazerieren des pulverisierten Pflanzenmaterials mit Lösungsmitteln, und
- Extrahieren des mazerierten Pflanzenmaterials.

2. Verfahren nach Anspruch 1, wobei der Fermentationsschritt eine Feststoff- oder Flüssigfermentation, vorzugsweise eine Feststofffermentation ist.

3. Verfahren nach einem der Ansprüche 1 und 2, wobei die Feststofffermentation mit einem oder mehreren der folgenden Mikroorganismen durchgeführt wird: Pilze, Bakterien, Hefen, Archaeen, Algen.

4. Verfahren nach einem der Ansprüche 1-3, wobei die im Fermentationsschritt verwendeten Bakterien vorzugsweise eines oder mehrere der folgenden sind: Gattung Pedicoccus, Gattung Lactococcus, Gattung Lactobacillus (z. B. *L. plantarum, L. brevis, L. casei, L. rhamnosus, L. curvatus, L. gasseri, L. pentosus, L. acidophilus*)*,* Bacillus pumilus, Bacillus subtilis, Trichoderma reesei, E faecium, P. acidilactici und P. pentosaceus.

5. Verfahren nach einem der Ansprüche 1 bis 4, wobei die für die Fermentation verwendeten Mikroorganismen in einem Verhältnis von 1:1 bis 1:1000 vorliegen, am meisten bevorzugt in einem Verhältnis von Mikrobe zu Cymbidium-Pflanzenmaterial von 1:50.

6. Verfahren nach einem der Ansprüche 1-5, wobei die Fermentationszeit zwischen 0,1 Stunde und 168 Stunden dauert, vorzugsweise 12-24 Stunden.

7. Verfahren nach einem der Ansprüche 1 - 6, wobei die verwendeten Lösungsmittel eines oder mehrere von Wasser, überkritischem CO₂ , Alkoholen, Ethanol, Methanol, Dichlormethan (DCM), Acetylacetat, Aceton und Pflanzenölen sind.

8. Verfahren nach einem der Ansprüche 1-7, wobei im Schritt der Beschallung ein Ultraschallgerät für 1 min-1440 min, vorzugsweise für 30-45 min, verwendet wird.

9. Verfahren nach einem der Ansprüche 1-8, wobei das Pflanzenmaterial nach dem Beschallungsschritt in 95% Ethanol für 1 h-168 h, vorzugsweise für 72 h bei Raumtemperatur mazeriert wird.

10. Verfahren nach einem der Ansprüche 1-9, wobei der Extraktionsschritt ein geeignetes Lösungsmittel verwendet, ausgewählt aus der Gruppe von Lösungsmitteln, umfassend Aceton, Kohlendioxid, Isopropanol oder t-Butanol und Ester von Essigsäure, vorzugsweise Ethylacetat, Alkohole, Ethanol B. Isopropylalkohol, Acetylacetat, Aceton, Pflanzenöl, und umfasst ferner die Schritte:
(a) Einbringen von *Cymbidium erythraeum-Material* in ein geeignetes Lösungsmittel, vorzugsweise 95 % Ethanol in Wasser, um eine Extraktion des Pflanzenmaterials zu erreichen;
(b) Trennen des flüssigen und festen Inhalts;
(c) Gewinnen einer ersten Fraktion aus den flüssigen Inhalten durch Filtration des in den flüssigen Inhalten vorhandenen Lösungsmittels;
(d) Einbringen der festen Inhaltsstoffe in ein organisches Lösungsmittel, ausgewählt aus der Gruppe von Lösungsmitteln, umfassend Aceton, Kohlendioxid, Isopropanol oder t-Butanol und Essigsäureester, vorzugsweise Ethylacetat, um eine Extraktion der verbleibenden Fraktion aus dem Pflanzenmaterial zu erreichen;
(e) Trennen des flüssigen und festen Inhalts;
(f) Gewinnen einer zweiten Fraktion durch Filtration des Lösungsmittels aus dem flüssigen Inhalt unter reduziertem Druck;
(g) Rückgewinnen des festen Inhalts und
h) lyophilisiert.

11. Zusammensetzung, erhalten durch das Verfahren nach einem der Ansprüche 1-10, **dadurch gekennzeichnet, dass** sie zur Behandlung oder Prophylaxe von Krankheiten beim Menschen verwendet wird.

12. Zusammensetzung nach Anspruch 11 zur Verwendung bei der Behandlung oder Prophylaxe von Magen-Darm-Erkrankungen, Krebs oder Hauterkrankungen bei Menschen, die mit einer beeinträchtigten oder verminderten Funktion der Darmmikrobiota und der Enzyme Chinonreduktase NQO1 und NQO2 in Zusammenhang stehen.

13. Zusammensetzung nach einem der Ansprüche 11-12 zur Verwendung bei der Behandlung oder Prophylaxe von Darmmikrobiota-Dysbiose

14. Zusammensetzung nach einem der Ansprüche 11-13 zur Verwendung bei der Behandlung oder Prophylaxe von Magen-Darm-Erkrankungen im Zusammenhang mit Darmmikrobiota-Dysbiose, wie Morbus Crohn, Colitis ulcerosa, Reizdarmsyndrom, Dysbiose der Magen-Darm-Mikrobiota, Adipositas, metabolisches Syndrom , Lebererkrankungen wie nichtalkoholische Steatohepatitis, nichtalkoholische Fettlebererkrankung und Zirrhose.

15. Zusammensetzung nach einem der Ansprüche 11- 13 zur Verwendung bei der Behandlung oder Prophylaxe von Krebs, insbesondere hepatozellulärem Karzinom, um die Aktivität der Chininreduktase NQO1 und NQO2 zu steigern.

16. Zusammensetzung nach einem der Ansprüche 11-13 zur Verwendung bei der Behandlung oder Prophylaxe von Hauterkrankungen im Zusammenhang mit Dysbiose der Darmmikrobiota und der Darm-Haut-Achse, wie Acne vulgaris, Rosacea.

17. Zusammensetzung nach einem der Ansprüche 11-12 zur Verwendung bei der Behandlung oder Prophylaxe von Hauterkrankungen im Zusammenhang mit einer verminderten oder beeinträchtigten Funktion der Enzyme Chininreduktase NQO1 und NOO2, insbesondere für beschleunigte oder chronologische Alterungsveränderungen der Haut wie Falten, Verlust der Festigkeit und Elastizität, erhöhte Talgproduktion und erhöhte Melaninpigmentierung.

18. Zusammensetzung zur Verwendung nach einem der Ansprüche 11-17, wobei der Bereich der verteilten Menge für die tägliche Dosis der Behandlung oder Prophylaxe zwischen 0,01 mg bis 10000 mg, vorzugsweise zwischen 1 mg-100 mg des 100%igen Produkts liegt eines Menschen, abhängig von der Störung und ihrer Schwere.

19. Zusammensetzung zur Verwendung nach einem der Ansprüche 11-17, wobei die Zusammensetzung einem Menschen in jeder geeigneten galenischen Form, wie Getränken, Sirupen, Kapseln, Tabletten, magensaftresistenten Tabletten, magensaftresistenten Kapseln, Suppositorien, Einläufen, verabreicht wird.

20. Zusammensetzung zur Verwendung nach einem der Ansprüche 11-19, wobei die Zusammensetzung ferner andere bioaktive Verbindungen umfasst, wie etwa: bioaktive Kohlenhydrate, Lipide, Aminosäuren, Proteine, Peptide, Oligoelemente, Enzyme, Pflanzengewebefaktoren, Enzyminhibitoren, Flavonoide, Thylakoide, Polyphenole und Xanthophylle.

21. Zusammensetzung zur Verwendung nach einem der Ansprüche 11- 20, wobei die Zusammensetzung ferner Wirkstoffe und Pflanzenextrakte wie Ascorbinsäure, Astaxanthin, Haematococcus-Algen, probiotische Substanzen, präbiotische Substanzen, postbiotische Substanzen, Omega-3-Fettsäuren, Fischöl, Planktonöl, Spirulina platensis Extrakt, Phycocyanin, Fucoxanthin, Coenzym Q10, Glutathion, Aminosäuren z.B. Cystein, Vitamine, Tocopherol, Lutein, Lycopin, Nikotinamid, Polyphenole umfasst.

## Revendications

1. Procédé de préparation d'une composition appropriée pour être utilisée dans le traitement ou la prophylaxie d'une maladie chez l'homme liée à une fonction altérée ou réduite du microbiote intestinal et de l'enzyme quinone réductase obtenue à partir de *Cymbidium eyrthraeum* fermenté comprenant un matériau végétal comprenant une plante ou des parties de plante de tous les types sauvages, hybrides et cultivars, trouvés dans la nature ou cultivés in vitro **caractérisé en ce que** le procédé comprend les étapes suivantes:
- le flétrissement de la matière végétale telle que des feuilles, des fleurs, des tiges, des graines, des cellules, des cellules souches et/ou une racine comprenant des tissus, des cellules et/ou des cellules souches des plantes ou des parties de plantes,
- la macération mécanique de la matière végétale flétrie,
- la fermentation de la matière végétale macérante par un procédé enzymatique, bactérien ou fongique,
- le séchage et pulvérisation de la matière végétale fermentée,
- la sonication et macération de la matière végétale pulvérisée avec des solvants, et
- l'extraction de la matière végétale macérée.

2. Procédé selon la revendication 1, dans lequel l'étape de fermentation est une fermentation solide ou liquide, de préférence une fermentation solide.

3. Procédé selon l'une quelconque des revendications 1 et 2, dans lequel la fermentation solide est effectuée avec un ou plusieurs des microorganismes suivants : champignons, bactéries, levures, archées, algues.

4. Procédé selon l'une quelconque des revendications 1 à 3, dans lequel les bactéries utilisées dans l'étape de fermentation sont de préférence une ou plusieurs des suivantes : genre Pedicoccus, genre Lactococcus, genre Lactobacillus (par exemple *L. plantarum, L. brevis, L. casei, L. rhamnosus, L. curvatus, L. gasseri, L. pentosus* , *L. acidophilus*)*,* Bacillus pumilus, Bacillus subtilis, Trichoderma reesei, E faecium, P.acidilactici et P. pentosaceus.

5. Procédé selon l'une quelconque des revendications 1 à 4, dans lequel les micro-organismes utilisés pour la fermentation sont dans un rapport de 1:1 à 1:1000, le plus préférablement un rapport de 1:50 entre le microbe et la matière végétale Cymbidium.

6. Procédé selon l'une quelconque des revendications 1 à 5, dans lequel le temps de fermentation prend entre 0,1 heure et 168 heures, de préférence 12 à 24 heures.

7. Procédé selon l'une quelconque des revendications 1 à 6, dans lequel les solvants utilisés sont un ou plusieurs parmi l'eau, le CO₂ supercritique, les alcools, l'éthanol, le méthanol, le dichlorméthane (DCM), l'acétate d'acétyle, l'acétone et les huiles végétales.

8. Procédé selon l'une quelconque des revendications 1 à 7, dans lequel, dans l'étape de sonication, un appareil à ultrasons est utilisé pendant 1 min à 1440 min, de préférence pendant 30 à 45 minutes.

9. Procédé selon l'une quelconque des revendications 1 à 8, dans lequel le matériel végétal après l'étape de sonication est macéré dans de l'éthanol à 95 % pendant 1 h à 168 h, de préférence pendant 72 h à température ambiante.

10. Procédé selon l'une quelconque des revendications 1 à 9, dans lequel l'étape d'extraction utilise un solvant approprié choisi dans le groupe de solvants comprenant l'acétone, le dioxyde de carbone, l'isopropanol ou le t-butanol et les esters d'acide acétique, de préférence l'acétate d'éthyle, les alcools, l'éthanol, alcool isopropylique, acétate d'acétyle, acétone, huile végétale, et comprend en outre les étapes suivante:
(a) le placement de la matière de *Cymbidium erythraeum* dans un solvant approprié, de préférence de l'éthanol à 95 % dans de l'eau pour réaliser l'extraction de la matière végétale;
(b) la séparation des contenus liquide et solide;
(c) la récupération d'une première fraction du contenu liquide par filtration du solvant présent dans le contenu liquide;
(d) le placement du contenu solide dans un solvant organique choisi dans le groupe de solvants comprenant l'acétone, le dioxyde de carbone, l'isopropanol ou le t-butanol et les esters de l'acide acétique, de préférence l'acétate d'éthyle pour réaliser l'extraction de la fraction restante de la matière végétale;
(e) la séparation des contenus liquide et solide;
(f) la récupération d'une seconde fraction par filtration du solvant à partir du contenu liquide sous pression réduite;
(g) la récupération du contenu solide, et
h) lyophilisé.

11. Composition obtenue par le procédé selon l'une quelconque des revendications 1 à 10, **caractérisée en ce qu'elle** est utilisée dans le traitement ou la prophylaxie de maladies chez l'homme.

12. Composition selon la revendication 11 pour être utilisée dans le traitement ou la prophylaxie des troubles gastro-intestinaux, du cancer ou des troubles cutanés chez l'homme liés à une fonction altérée ou réduite du microbiote intestinal et de l'enzyme quinone réductase NQO1 et NOO2.

13. Composition selon l'une quelconque des revendications 11 à 12 pour être utilisée dans le traitement ou la prophylaxie de la dysbiose du microbiote intestinal.

14. Composition selon l'une quelconque des revendications 11 à 13 pour être utilisée dans le traitement ou la prophylaxie des troubles gastro-intestinaux liés à la dysbiose du microbiote intestinal tels que la maladie de Crohn, la colite ulcéreuse, le syndrome du côlon irritable, la dysbiose du microbiote gastro-intestinal, l'obésité, le syndrome métabolique, des affections hépatiques telles que la stéatohépatite non alcoolique, la stéatose hépatique non alcoolique et la cirrhose.

15. Composition selon l'une quelconque des revendications 11 à 13 pour être utilisée dans le traitement ou la prophylaxie du cancer, en particulier du carcinome hépatocellulaire, pour stimuler l'activité de la quinine réductase NQO1 et NOO2.

16. Composition selon l'une quelconque des revendications 11 à 13 pour être utilisée dans le traitement ou la prophylaxie des troubles cutanés liés à la dysbiose du microbiote intestinal et à l'axe intestin-peau, tels que l'acné vulgaire, la rosacée.

17. Composition selon l'une quelconque des revendications 11 à 12 pour être utilisée dans le traitement ou la prophylaxie des troubles cutanés liés à une fonction diminuée ou altérée de l'enzyme quinine réductase NQO1 et NOO2, spécifiquement pour les changements accélérés ou chronologiques du vieillissement de la peau tels que les rides, perte de fermeté et d'élasticité, augmentation de la production de sébum et augmentation de la pigmentation de la mélanine.

18. Composition pour être utilisée selon l'une quelconque des revendications 11 à 17, dans laquelle la fourchette de la quantité distribuée pour la dose quotidienne du traitement ou de la prophylaxie est comprise entre 0,01 mg et 10 000 mg, de préférence entre 1 mg et 100 mg de produit à 100 % pour un être humain, selon le trouble et sa gravité.

19. Composition pour être utilisée selon l'une quelconque des revendications 11 à 17, dans laquelle la composition est administrée à un être humain sous toute forme galénique appropriée telle que boissons, sirops, gélules, comprimés, comprimés à enrobage entérique, gélules à enrobage entérique, suppositoires, lavements.

20. Composition pour être utilisée selon l'une quelconque des revendications 11 à 19, dans laquelle la composition comprend en outre d'autres composés bioactifs tels que: des glucides bioactifs, des lipides, des acides aminés, des protéines, des peptides, des oligo-éléments, des enzymes, des facteurs tissulaires végétaux, des inhibiteurs d'enzymes, flavonoïdes, thylacoïdes, polyphénols et xanthophylles.

21. Composition pour être utilisée selon l'une quelconque des revendications 11 à 20, dans laquelle la composition comprend en outre des ingrédients actifs et un extrait végétal tel que l'acide ascorbique, l'astaxanthine, les algues hématocoques, les substances probiotiques, les substances prébiotiques, les substances postbiotiques, les acides gras oméga-3, l'huile de poisson, huile de plancton, extrait de spirulina platensis, phycocyanine, fucoxanthine, coenzyme Q10, gluathion, acides aminés par ex. cystéine, vitamines, tocophérol, lutéine, lycopène, amide de nicotine, polyphénols.
